# EUROPEAN PATENT APPLICATION

(11) **EP 4 706 713 A1**
(43) Date of publication of application: **11.03.2026**
(21) Application number: 25200226.6
(22) Date of filing: 04.09.2025
(51) Int. Cl.: A61M 16/00, A61M 16/10, A61M 16/12

(54) **OXYGEN THERAPY APPARATUS**

(30) Priority: 10.09.2024 KR 20240123324
(71) Applicant: Seoil Pacific Corp., Seoul 08389 (KR)
(72) Inventor: KIM, Kye Cheol, 10391 Gyeonggi-do (KR)
(74) Representative: Witte, Weller & Partner Patentanwälte mbB

(57) **Abstract**

An oxygen therapy apparatus (10) comprises: a blower (100) that delivers introduced oxygen and external air; a mixing chamber (200) where the oxygen and air delivered by the blower are mixed; a guide tube (810, 820) that provides gas mixed in the mixing chamber unit to a patient; and an oxygen sensor (830) that measures concentration of oxygen in the gas mixed in the mixing chamber. The mixing chamber is divided into a first layer and a second layer, and the oxygen and air delivered by the blower move and are mixed in order through a first mixing chamber (240) of the first layer, a second mixing chamber (250) of the second layer, a third mixing chamber (260) of the first layer, and a fourth mixing chamber (270) of the first layer before being provided to a user.

## Description

### BACKGROUND

### Technical Field

The present disclosure generally relates to an oxygen therapy apparatus.

### Description of Related Art

Many people suffer from respiratory and bronchial disorders caused by asthma, pollen allergies, pollutants, and other factors. Patients may experience rapid worsening of symptoms such as coughing, dyspnea, wheezing, and chest tightness due to environmental factors, which can impair the smooth supply of oxygen essential for patient survival.

In such cases, it is required to isolate patients from environments containing allergens, pollutants, and other causes. However, respiratory difficulties may persist even after isolation, and in such cases, it is necessary to provide air and oxygen to patients through respiration.

Conventional oxygen therapy devices have a simple structure that simultaneously introduces air and oxygen and simply provides the introduced oxygen and air to patients. Therefore, there was a difficulty in that oxygen and air were provided to users without being sufficiently mixed.

### SUMMARY

According to one aspect of the present disclosure, an oxygen therapy apparatus comprises: a blower that delivers introduced oxygen and external air; a mixing chamber where the oxygen and air delivered by the blower are mixed; a guide tube that provides the gas mixed in the mixing chamber unit to a patient; and an oxygen sensor that measures the concentration of oxygen in the gas mixed in the mixing chamber unit, wherein the mixing chamber unit is divided into a first layer and a second layer, and the oxygen and air delivered by the blower move and are mixed in the order of a first mixing chamber of the first layer, a second mixing chamber of the second layer, a third mixing chamber of the first layer, and a fourth mixing chamber of the first layer before being provided to a user.

According to another aspect, the blower delivers the oxygen and external air through an inlet formed in the first mixing chamber.

According to another aspect, in the mixing chamber, the first layer and the second layer are partitioned by a partition member, and the first to fourth mixing chambers are partitioned by the partition member. In this aspect, the partition member is formed with: a connection passage connecting the first mixing chamber of the first layer and the second mixing chamber of the second layer; a connection passage connecting the second mixing chamber of the second layer and the third mixing chamber of the first layer; and a connection passage connecting the third mixing chamber of the first layer and the fourth mixing chamber of the first layer. Also in this aspect, the mixing chamber unit further includes a buffer space of the second layer, and the gas mixed in the buffer space is delivered to the user. In this aspect, the partition member further includes a blocking member that separates the third mixing chamber and the fourth mixing chamber, and the blocking member is formed shorter than the height of the first layer to form a passage through which gas moves from the third mixing chamber to the fourth mixing chamber.

According to another aspect, the oxygen sensor is connected to the fourth mixing chamber through a hole penetrating the outer peripheral wall of the mixing chamber unit and measures the concentration of oxygen in the mixed gas in the fourth mixing chamber.

According to another aspect, the oxygen therapy apparatus further comprises a humidification unit that humidifies the air mixed with the oxygen and air, including: a heating plate; a water tank; a first guide tube connecting the mixing chamber unit and the water tank; and a second guide tube connecting the water tank and a user. In this aspect, the oxygen therapy apparatus further comprises a heating member that heats the second guide tube.

According to another aspect, the oxygen therapy apparatus further comprises a filter that filters foreign substances from the external air.

According to another aspect, the oxygen therapy apparatus further comprises a muffler member that reduces noise by lowering the flow velocity of introduced air. In this aspect, the muffler member includes: an ascending guide path that moves the introduced air upward; and a parallel flow chamber that moves the ascending air in parallel, wherein the flow cross-sectional area of the parallel flow chamber is larger than the cross-sectional area of the ascending guide path. In this aspect, the muffler member includes: a vertical flow member in which the ascending guide path is formed; a sealing member that seals the vertical flow member; a cover member in which the parallel flow chamber is formed; and a sealing member that seals the cover member.

According to another aspect, the air that has passed through the muffler member is introduced into the blower together with the introduced oxygen.

### BRIEF DESCRIPTION OF THE DRAWINGS

FIG. 1 is a perspective view schematically showing the front of an oxygen therapy apparatus according to the present embodiment.
FIG. 2 is a perspective view schematically showing the rear of the oxygen therapy apparatus according to the present embodiment.
FIG. 3 is a drawing schematically illustrating a state where a blower and a mixing chamber unit are mounted in a second housing.
FIG. 4 is a drawing illustrating the flow of air with arrows in a state where a muffler 400 is mounted on the blower 100.
FIG. 5 is a drawing illustrating a state where a partition member and a mixing chamber unit are combined to form first to fifth mixing chambers.
FIG. 6 is a drawing illustrating a partition member.

### DETAILED DESCRIPTION OF THE EMBODIMENTS

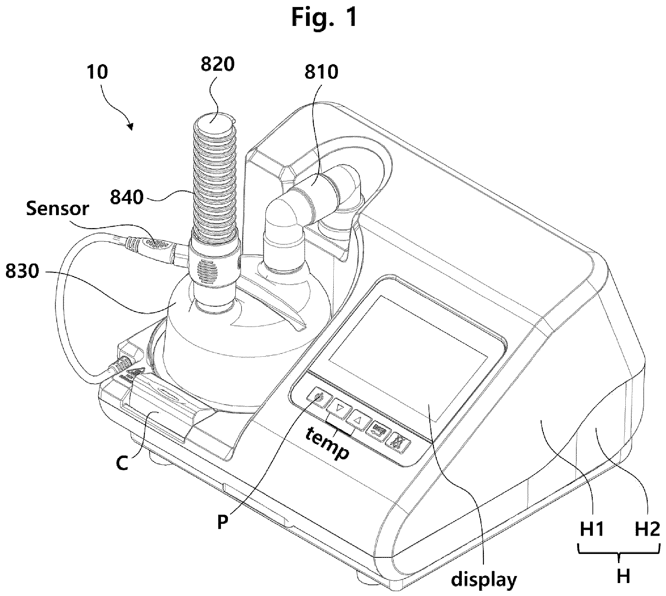

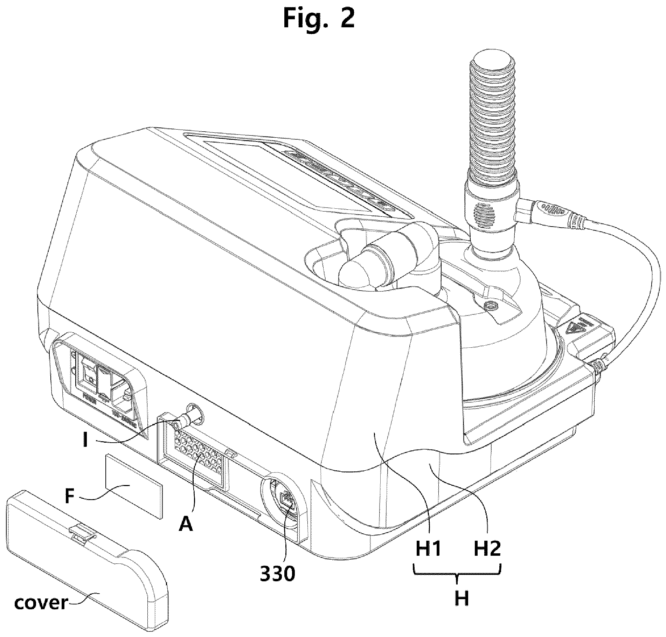

Hereinafter, the present embodiment will be described with reference to the accompanying drawings. FIG. 1 is a perspective view schematically showing the front of an oxygen therapy apparatus 10 according to the present embodiment, and FIG. 2 is a perspective view schematically showing the rear of the oxygen therapy apparatus 10 according to the present embodiment. Referring to FIGS. 1 and 2, the oxygen therapy apparatus 10 according to the present embodiment includes a housing H in which an upper housing H1 and a lower housing H2 are combined.

On the front of the oxygen therapy apparatus 10, there are located a power button P that controls ON/OFF of the oxygen therapy apparatus 10 of the present embodiment, a temperature control switch (temp) that adjusts the temperature of air provided to users, and a display that shows operation information of the oxygen therapy apparatus 10. The oxygen therapy apparatus 10 may further include a humidification unit that humidifies gas mixed with oxygen and air and provides the humidified gas to patients through a second guide tube 820.

The humidification unit humidifies the gas provided by the first guide tube 810 and provides it to the second guide tube 820, and may include a water tank 830, a heating member (not shown) that heats the water tank 830, and a clamp C that fixes the water tank 830.

Additionally, the oxygen therapy apparatus 10 may further include a heating wire 840 that is connected to the end of a second guide tube that provides humidified air to users and heats the second guide tube 820. When the temperature of the gas provided to users through the second guide tube 820 drops below the condensation point, condensation may occur inside the second guide tube 820. The oxygen therapy apparatus 10 may heat the second guide tube 820 through the heating wire 840 to prevent this. The oxygen therapy apparatus 10 may further include a sensor that detects the temperature of air provided to users and may display the detected air temperature through the display.

On the rear of the oxygen therapy apparatus 10, there are located an oxygen inlet I connected to an oxygen supply source (not shown) such as an oxygen tank through which oxygen is provided, and an air inlet A through which air is introduced. A filter F may be located in front of the air inlet A to remove foreign substances such as dust. On the rear of the oxygen therapy apparatus 10, there may be located a communication connector 330 that is connected to an oxygen sensor 300 and outputs the oxygen concentration measured by the oxygen sensor 300. The oxygen concentration measured by the oxygen sensor 300 is directly provided to a control unit (not shown) and output to the display, but when there is a need to transmit oxygen concentration data externally, it can be connected to the communication connector 330 to determine the oxygen concentration of gas provided to patients. The communication connector 330 and the air inlet A with the filter F positioned in front can be covered with a cover member to prevent the introduction of foreign substances.

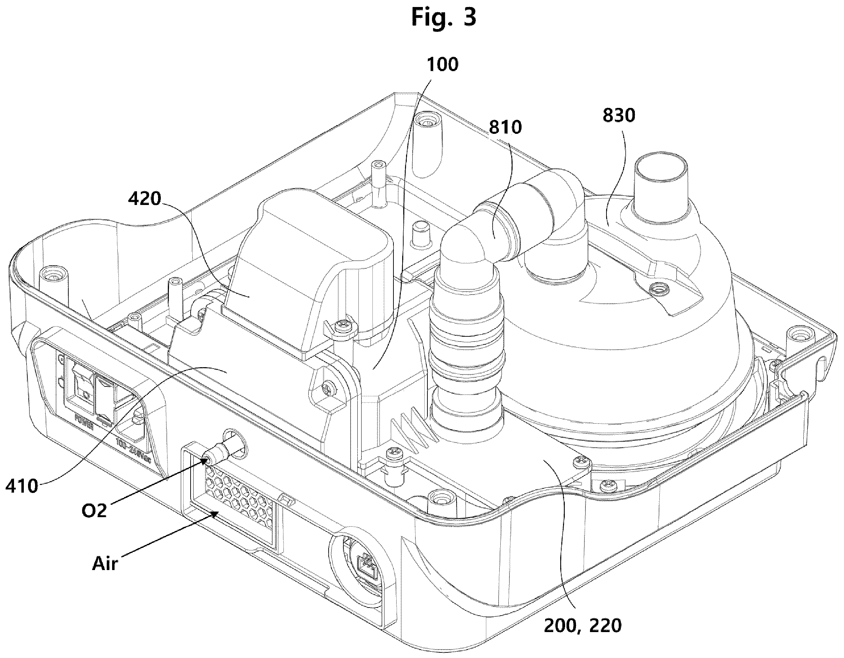

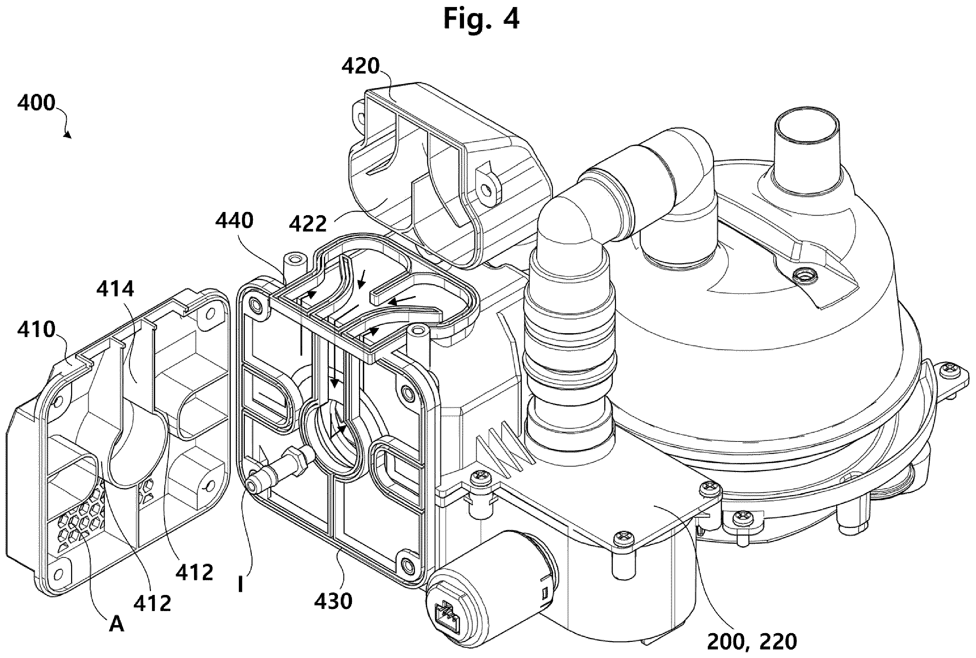

FIG. 3 is a drawing schematically illustrating a state where the blower 100 and mixing chamber unit 200 are mounted in the second housing H2. FIG. 3 shows a state where a vertical movement member 410 and cover member 420 of a muffler member are positioned on the blower 100, and the mixing chamber unit 200 is shown covered with a chamber cover 220. FIG. 4 shows the mixing chamber unit 200 with the blower 100 and chamber cover 220 removed.

FIG. 4 is a drawing illustrating the flow of air with arrows in a state where a muffler 400 is mounted on the blower 100. Referring to FIGS. 3 and 4, the muffler includes a vertical flow member 410 including an ascending guide path 412 that moves air introduced through the air inlet A upward, and a cover member 420 including a parallel flow chamber 422 that moves the ascending air in parallel. The vertical movement member 410 includes a descending guide path 414 that lowers the air provided from the parallel movement chamber 422 and provides it to the blower.

Air introduced through the air inlet A ascends through the ascending guide path 412 formed in the vertical flow member 410. The ascending air enters the parallel movement chamber 422 where air moves in parallel. As shown, the cross-sectional area through which air flows in the parallel movement chamber 422 is larger than the cross-sectional area through which air flows in the ascending guide path 412. Therefore, the speed at which the introduced air flows is reduced, and noise generated from air movement is reduced accordingly.

The parallel movement chamber 422 is connected to the descending guide path 414 of the vertical flow member 410. Therefore, air flowing out from the parallel movement chamber 422 descends through the descending guide path 414 and proceeds to the inlet of the blower 100 together with oxygen introduced through the oxygen inlet I.

In the illustrated embodiment, the ascending guide path 412 included in the vertical flow member 410 forms two branched paths with a descending guide path 424 positioned in the center between them. However, this is merely an embodiment, and the ascending guide path may be formed as one path or three or more paths, and the descending guide path may form multiple paths.

Additionally, the ascending guide path 412 and descending guide path 414 may be partitioned by partition walls formed in the vertical movement member 410 and may be sealed with the outer wall of the blower 100 and sealing members 430 such as rubber packing.

In the illustrated embodiment, the parallel movement chamber 422 may be connected to two ascending guide paths 412 and may be connected to the descending guide path 424 through a portion where the paths of the two ascending guide paths 412 merge.

In one embodiment, the parallel movement chamber 422 includes a chamber connected to two ascending guide paths and a chamber connected to a descending guide path, and each chamber may be partitioned by partition walls formed in the cover member and may be sealed with the outer wall of the blower 100 and sealing members 440 such as rubber packing.

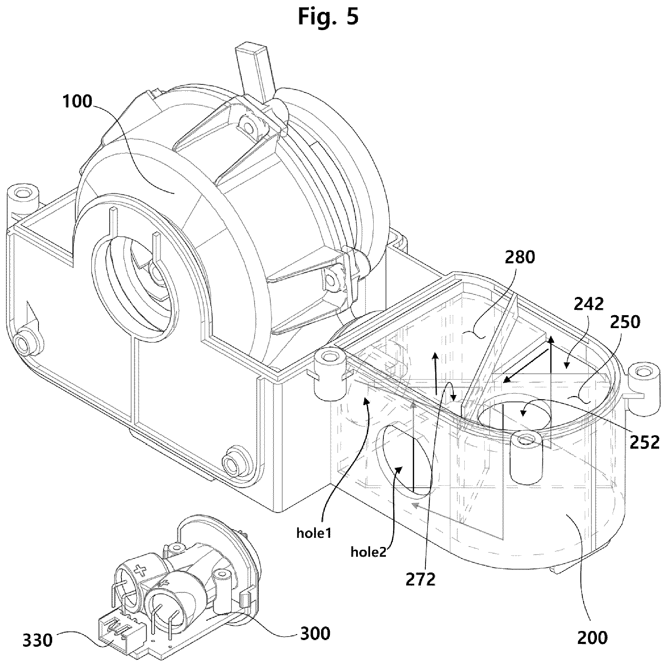

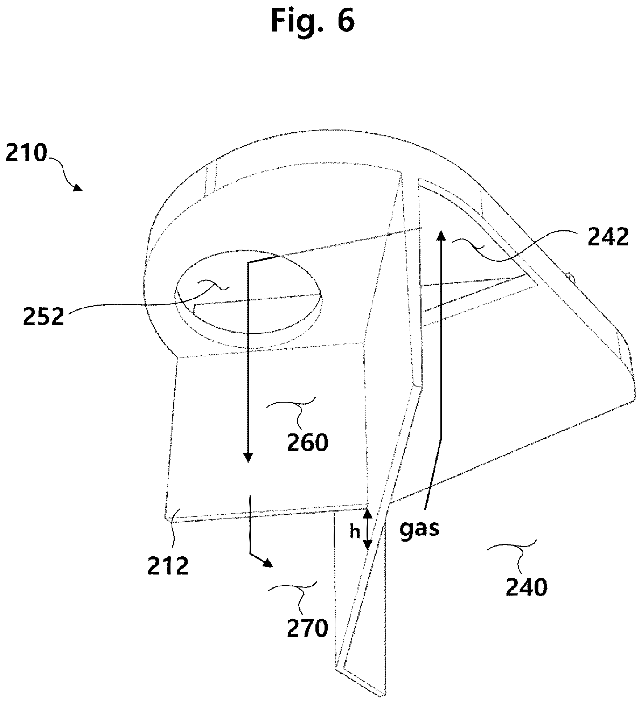

FIG. 5 is a drawing illustrating a state where the partition member 210 and mixing chamber unit 200 are combined to form first to fifth mixing chambers, and FIG. 6 is a drawing illustrating the partition member 210. In the embodiment illustrated in FIGS. 3 and 5, the chamber cover 220 covers the second layer of the mixing chamber unit 200 to isolate the second mixing chamber 250 and buffer space 280 from the outside. In one embodiment, the chamber cover 220 may isolate the second mixing chamber 250 and buffer space 280 from the outside using sealing members such as rubber packing.

Referring to FIGS. 1 to 6, oxygen O2 and air (Air) are introduced into the blower 100. Although oxygen O2 and air (Air) are introduced into the blower 100, they are not completely homogeneously mixed due to temperature differences, density differences, and other factors. When oxygen concentration is measured for incompletely mixed air, it is measured inaccurately according to gas flow.

In the present embodiment, oxygen O2 and air (Air) introduced into the blower 100 are introduced into the first mixing chamber 250 through a blower connection hole (hole 1) formed in the outer peripheral wall of the mixing chamber 200. The partition member 210 combines with the mixing chamber unit 200 and partitions the internal space of the mixing chamber unit 200 to form a first mixing chamber 240, second mixing chamber 250, third mixing chamber 260, fourth mixing chamber 270, and buffer space 280.

Hereinafter, the flow of air provided by the blower 100 will be described with reference to FIGS. 5 and 6. Gas in which oxygen and air provided by the blower 100 are non-homogeneously mixed is introduced into the first mixing chamber 240 through the blower connection hole (hole1) formed in the outer peripheral wall. The mixed gas of introduced oxygen and air stays in the first mixing chamber 240 formed on the first layer of the mixing chamber unit 200, mixes while staying, and ascends to the second mixing chamber 250 formed on the second layer of the mixing chamber unit 200 through the first connection passage 242 of the partition member 210.

The gas is mixed and blended in the second mixing chamber 250 and descends to the third mixing chamber 260 formed on the first layer of the mixing chamber unit 200 through the second connection passage 252 of the partition member 210. As shown, the gas mixed with air and oxygen ascends through the first connection passage 242 and descends to the third mixing chamber 260 through the second connection passage 252, gradually resolving non-homogeneity due to temperature and density differences to be mixed with high homogeneity.

In the third mixing chamber 260, the blocking member 212 that partitions the third mixing chamber 260 and fourth mixing chamber 270 is formed shorter by height h compared to the height of the first layer. This creates a passage through which gas moves to the fourth mixing chamber 270 without completely blocking the third mixing chamber 260.

Gas enters the fourth mixing chamber 270 through the passage formed at the bottom of the third mixing chamber 260. Gas provided through the blower 100 is mixed while ascending from the first mixing chamber 240 to the second mixing chamber 250, mixed while descending from the second mixing chamber 250 to the third mixing chamber 260, and mixed while proceeding to the fourth mixing chamber 280 through the passage at the bottom of the third mixing chamber 260, resulting in mixing without non-homogeneity due to temperature and density differences of the gas.

An oxygen sensor connection hole (hole2) is formed in the outer peripheral wall of the mixing chamber unit 220 and connected to the oxygen sensor 300. In one embodiment, the oxygen sensor 300 may be an ultrasonic oxygen sensor and may include an ultrasonic transmission module and an ultrasonic reception module as a pair to measure oxygen concentration. As described above, if oxygen and air are not homogeneously mixed, the speed of ultrasound changes non-ideally within the gas, preventing accurate measurement of oxygen concentration.

However, according to the present embodiment, gas is mixed to resolve non-homogeneity due to temperature and density differences of the gas, providing the advantage of being able to measure oxygen concentration with high accuracy. As described above, the oxygen sensor 300 is provided to a control unit (not shown), and the control unit can display the measured oxygen concentration to users through the display.

Gas mixed in the fourth mixing chamber 270 ascends to the buffer space 280 of the second layer of the mixing chamber unit 200 through the third connection passage 272, and the mixed gas located in the buffer space 280 proceeds to the water tank 830 through the aforementioned first guide tube 810.

The mixed gas that has entered the water tank 830 is provided to patients through the second guide tube 820 together with water vapor formed by a heating member (not shown) heating the water tank 830. Additionally, since water vapor may condense when gas containing water vapor is cooled through the path provided to patients, condensation is prevented by heating through heating lines 830.

Although described with reference to the embodiment shown in the drawings to help understand the present invention, this is an embodiment for implementation and is merely exemplary. Those skilled in the art will understand that various modifications and equivalent other embodiments are possible therefrom. Therefore, the true technical protection scope of the present invention should be determined by the appended patent claims.

## Claims

1. An oxygen therapy apparatus(10) comprising:
a blower(100) that delivers introduced oxygen and external air;
a mixing chamber(200) where the oxygen and air delivered by the blower are mixed;
a guide tube(810, 820) that provides gas mixed in the mixing chamber unit to a patient;
an oxygen sensor(300) that measures concentration of oxygen in the gas mixed in the mixing chamber unit(200),
wherein the mixing chamber unit(200) is divided into a first layer and a second layer, and
the oxygen and air delivered by the blower move and are mixed in order of a first mixing chamber(240) of the first layer, a second mixing chamber(250) of the second layer, a third mixing chamber(260) of the first layer, and a fourth mixing chamber(270) of the first layer before being provided to a user.

2. The oxygen therapy apparatus of claim 1, wherein the blower delivers the oxygen and external air through an inlet formed in the first mixing chamber.

3. The oxygen therapy apparatus of claim 1, wherein in the mixing chamber, the first layer and the second layer are partitioned by a partition member, and the first to fourth mixing chambers are partitioned by the partition member.

4. The oxygen therapy apparatus of claim 3, wherein the partition member is formed with: a connection passage connecting the first mixing chamber of the first layer and the second mixing chamber of the second layer; a connection passage connecting the second mixing chamber of the second layer and the third mixing chamber of the first layer; and a connection passage connecting the third mixing chamber of the first layer and the fourth mixing chamber of the first layer.

5. The oxygen therapy apparatus of claim 4, wherein the mixing chamber unit further comprises a buffer space of the second layer, and gas mixed in the buffer space is delivered to the user.

6. The oxygen therapy apparatus of claim 1, wherein the oxygen sensor is connected to the fourth mixing chamber through a hole penetrating an outer peripheral wall of the mixing chamber unit and measures concentration of oxygen in the mixed gas in the fourth mixing chamber.

7. The oxygen therapy apparatus of claim 1, further comprising a humidification unit that humidifies air mixed with the oxygen and air, the humidification unit including: a heating plate; a water tank; a first guide tube connecting the mixing chamber unit and the water tank; and a second guide tube connecting the water tank and a user.

8. The oxygen therapy apparatus of claim 7, further comprising a heating member that heats the second guide tube.

9. The oxygen therapy apparatus of claim 4, wherein the partition member further comprises a blocking member that separates the third mixing chamber and the fourth mixing chamber, and the blocking member is formed shorter than a height of the first layer to form a passage through which gas moves from the third mixing chamber to the fourth mixing chamber.

10. The oxygen therapy apparatus of claim 1, further comprising a filter that filters foreign substances from the external air.

11. The oxygen therapy apparatus of claim 1, further comprising a muffler member that reduces noise by lowering flow velocity of introduced air.

12. The oxygen therapy apparatus of claim 11, wherein the muffler member comprises: an ascending guide path that moves the introduced air upward; and a parallel flow chamber that moves ascending air in parallel, wherein a flow cross-sectional area of the parallel flow chamber is larger than a cross-sectional area of the ascending guide path.

13. The oxygen therapy apparatus of claim 12, wherein the muffler member comprises: a vertical flow member in which the ascending guide path is formed; a sealing member that seals the vertical flow member; a cover member in which the parallel flow chamber is formed; and a sealing member that seals the cover member.

14. The oxygen therapy apparatus of claim 1, wherein air that has passed through the muffler member is introduced into the blower together with the introduced oxygen.
